Europäisches Patentamt

European Patent Office

Office européen des brevets

(19)

(11) Veröffentlichungsnummer : **0 409 053 B1**

## (12) EUROPÄISCHE PATENTSCHRIFT

(45) Veröffentlichungstag der Patentschrift :
**29.09.93 Patentblatt 93/39**

(51) Int. Cl.$^5$ : **C07K 3/28,** C07K 3/02

(21) Anmeldenummer : **90113187.0**

(22) Anmeldetag : **11.07.90**

(54) **Verfahren zur Reinigung von Annexinen.**

(30) Priorität : **15.07.89 DE 3923501**

(43) Veröffentlichungstag der Anmeldung :
**23.01.91 Patentblatt 91/04**

(45) Bekanntmachung des Hinweises auf die
Patenterteilung :
**29.09.93 Patentblatt 93/39**

(84) Benannte Vertragsstaaten :
**AT BE CH DE DK ES FR GB GR IT LI LU NL SE**

(56) Entgegenhaltungen :
**EP-A- 0 181 465**

(56) Entgegenhaltungen :
**THE JOURNAL OF BIOLOGICAL CHEMISTRY,
Band 264, Nr. 24, 25. August 1989, Seiten
14463-14470; M.A. KAETZEL et al.:
"Differential tissue expression of three 35-
kDa annexin calcium-dependent phospholi-
pid-binding proteins"**

(73) Patentinhaber : **BOEHRINGER INGELHEIM
INTERNATIONAL G.M.B.H.
D-55216 Ingelheim (DE)**

(72) Erfinder : **Reutelingsperger, Christiaan, Dr.
Looiersgracht 17
NL-6211 JK Maastricht (NL)**
Erfinder : **Bodo, Gerhard, Prof.-Dr.
Belghoferstrasse 27/5
A-1120 Wien (AT)**

EP 0 409 053 B1

**Beschreibung**

In den meisten Säugetieren existieren Proteine, die blutgerinnungshemmende Eigenschaften aufweisen. Diese Proteine können in drei Gruppen eingeteilt werden, wobei die Einteilung auf den unterschiedlichen Wirkmechanismen beruht.

1. Proteine, die mit dem Gerinnungsfaktor einen Komplex bilden und dadurch den Gerinnungsfaktor unwirksam machen. Dazu gehören die Proteine:

    a) Antithrombin-III (Thromb.Res.5, 439-452 (1974))

    b) $\alpha_1$-Protease Inhibitor (Ann. Rev. Biochem. 52, 655-709 (1983))

    c) $\alpha_2$-Makroglobulin (Ann. Rev. Biochem.. 52, 655-709 (1983))

    d) $C_1$-Inhibitor (Biochemistry 20, 2738-2743 (1981))

    e) Protease Nexin (J. Biol. Chem. 258, 10439-10444, (1983)).

2. Proteine, die einen Gerinnungsfaktor proteolytisch zerschneiden und dadurch den Gerinnungsfaktor desaktivieren. Als einziges Protein dieser Art ist bisher Protein C beschrieben (J. Biol. Chem. 251, 355-363, (1976)).

3. Proteine, die die negativ geladenen Phospholipide abschirmen und/oder hydrolisieren, so daß die Phospholipid-abhängigen Reaktionen des Blutgerinnungsmechanismus gehemmt werden. Bisher sind nur Phospholipasen, die aus verschiedenen Schlangengiftsorten isoliert wurden, beschrieben worden (Eur. J. Biochem. 112, 25-32 (1980)).

Das stufenweise ablaufende Gerinnungssystem ist in den letzten Jahren intensiv untersucht worden. Es wird als ein sich verstärkendes Mehrstufensystem verschiedener miteinander verbundener, proteolytischer Reaktionen verstanden, bei dem ein enzym ein Zymogen in die aktive Form umsetzt (vgl. Jackson C.M., Nemerson Y., Ann. Rev. Biochem. 49, 765-811, (1980)). Die Geschwindigkeit dieser Reaktion wird in entscheidender Weise durch die Anwesenheit von Phospholipiden und anderer Kofaktoren wie Faktor $V_a$ und Faktor $VIII_a$ vergrößert. In vivo werden die Prokoagulationsreaktionen durch verschiedene Inhibitationsmechanismen geregelt, die einem explosiv thrombotischen Trauma nach einer geringen Aktivierung der Gerinnungskaskade vorbeugen.

Die Antigerinnungsmechanismen können wie folgt eingeteilt werden (Rosenberg, R.D., Rosenberg, J.S., J. Clin. Invest. 74, 1-6 (1984)):

1. Der Serin-Protease-Faktor $X_a$ und Thrombin werden infolge ihrer Bindung an Antithrombin III bzw. an den Antithrombin/Heparin-Komplex inaktiviert. Sowohl die Prothrombin-Aktivierung als auch die Bildung von Fibrin kann auf diese Weise gehemmt werden. Neben Anti-thrombin III gibt es noch verschiedene andere Plasma-Protease-Inhibitoren wie beispielsweise $\alpha_2$-Makroglobulin und Antitrypsin, deren Wirkung zeitabhängig ist.

2. Die Entdeckung des Protein C's führte zur Aufdeckung eines weiteren Antigerinnungsmechanismus. Ist das Protein C einmal aktiviert worden, wirkt es durch die selektive Proteolyse der Proteinkofaktoren Faktor $V_a$ und $VIII_a$, durch die die Prothrombinase und das Enzym, das den Faktor X umsetzt, inaktiviert werden, als Antikoagulanz.

3. Plasmin spaltet monomeres Fibrin 1, ein Produkt der Einwirkung von Thrombin auf Fibrinogen, wodurch der Bildung eines unlöslichen Fibrins vorgebeugt wird (Nosssel, H.L., Nature, 291, 165-167 (1981)).

Von den oben genannten, in den Gerinnungsprozeß eingreifenden körpereigenen Proteinen ist z.Zt. nur das Antithrombin III in klinischem Einsatz. Als erheblicher Nachteil hat sich jedoch die bei der Anwendung dieses Proteins auftretende Erhöhung der Blutungsneigung herausgestellt.

Alle bisher als Antikoagulantien eingesetzten Mittel, ob körpereigener oder synthetischer Natur machen in irgendeiner Weise die Gerinnungsfaktoren unwirksam und führen dadurch zu Nebenwirkungen, die sich nachteilig auf den Gerinnungsprozeß auswirken können.

Überraschenderweise konnten nun neben diesen Proteinen weitere körpereigene Stoffe isoliert werden, die zwar die gewünschten blutgerinnungshemmenden Eigenschaften unter besonderen Bedingungen zeigen, hierbei aber die Blutungsgefahr nicht erhöhen. Bei größeren Blutungen verlieren diese Proteine ihre blutgerinnungshemmenden Eigenschaften und stören dadurch bei deren Verwendung nicht die in solchen Fällen überlebensnotwendigen Gerinnungsprozesse. Da sie erstmals isoliert wurden aus stark vaskularisiertem Gewebe, wurden sie "vascular anticoagulating proteins", VAC genannt.

Die aus stark vaskularisierten Geweben wie Nabelschnurgefäßen und Placenta isolierten Proteine weisen Molekulargewichte von ca. $70 \times 10^3$, ca. $60 \times 10^3$, ca. $34 \times 10^3$ und ca. $32 \times 10^3$ auf, von denen die Substanzen mit den Molekulargewichten 34 respektive $32 \times 10^3$ aus einer einzigen Polypeptidkette bestehen. Die genaue biochemische Charakterisierung dieser Proteine, sowie deren Isolierung und Reinigung findet sich in der EP-A-0 181 465.

Proteine mit VAC Aktivitität stellen natürliche Blutgerinnungshemmstoffe dar, die bei der Blutgerinnungskaskade an zwei Stellen eingreifen.

Das erste Mal inhibieren sie bei der durch die Faktoren IXa und VIIIa katalysierten Aktivierung des Faktors X zu Xa, das zweite Mal unterbinden sie die Spaltung von Prothrombin zu Thrombin, die durch die Faktoren Xa und Va vermittelt wird. Beiden Aktivierungsschritten ist gemeinsam, daß sie Calcium-Ionen und Phospholipide benötigen. Offenbar können VAC-Proteine ebenfalls mit Phospholipiden in Wechselwirkung treten, und durch diese Bindung die Aktivierungsschritte der Gerinnnungsfaktoren blockieren.

Wie sich mittlerweile gezeigt hat, gibt es eine ganze Familie, die wie VAC an Phospholipide in Calziumabhängiger Weise binden und mit Phospholipidoberflächen abhängigen Prozessen interferieren.

Zu dieser Familie, die auch Annexine genannt wird, gehören neben Lipocortin I, Calpactin I, Protein II, Lipocortin III, p67-Calelectrin auch IBC, PAP, PAP I, PP4, Endonexin II und Lipocortin V.

Die gemeinsamen strukturellen Merkmale der Annexine sind wahrscheinlich die Grundlagen für ihre ähnlichen $Ca^{2+}$ und Phospholipidbindungseigenschaften. Obwohl diese generelle Eigenschaft für alle Annexine gilt, besteht eine klare Individualität hinsichtlich ihrer Affinität zu $Ca^{2+}$ und zu den verschiedenen Phospholipidarten.

Die physiologischen Funktionen der Annexine betreffen membranassoziierte Prozesse. Der grundlegende Mechanismus der gerinnungshemmenden Wirkung des VAC wurde als eine Hemmung der katalytischen Kapazität der Phospholipide durch die Bindung des VAC an ihre Oberfläche erkannt, wodurch die Bildung des gerinnungsfördernden Komplexes an ihrer Oberfläche verhindert wird.

Auch andere Annexine können die Blutgerinnung hemmen, doch scheint VAC der effektivste Inhibitor zu sein.

Bindungsstudien haben gezeigt, daß sich VAC Calcium-abhängig mit prokoagulatorischen Phospholipiden reversibel assoziiert.

Auch andere bivalente Kationen aus der Reihe $Cd^{2+}$ $Zn^{2+}$, $Mn^{2+}$ und $Co^{2+}$ beeinflussen die Assoziation positiv, jedoch nicht in dem Maße wie $Ca^{2+}$.

Ihre Eigenschaften machen die Proteine zu interessanten, pharmakologisch äußerst wertvollen Wirkstoffen. Die gentechnische Methode, mit der es möglich war, VAC-Proteine herzustellen, wurde in der EPA 293 567 beschrieben, auf deren Lehre hier ausdrücklich Bezug genommen wird.

Aufgabe der vorliegenden Erfindung war, das in der EPA 293 567 beschriebene Verfahren zur Isolierung und Reinigung von VAC-Proteinen zu verbessern.

Bei dem aus der EPA 293 567 bekannten Verfahren wurde zur Isolierung und Reinigung der exprimierten Proteine die gefrorene Biomasse in einem geeigneten Lyse-Puffer suspendiert. Die Zellen wurden anschließend mechanisch, beispielsweise durch eine Manton-Gaulin Presse zerstört. Nach Zugabe eines Fällungsmittels für Nicht-Protein-Bestandteile, wie Polyethylenimin, wurden die festen Bestandteile beispielsweise durch Zentrifugation entfernt. Nach Ausfällung der Proteine, vorzugsweise durch Ammoniumsulfatfraktionierung, Auflösung des Präzipitates, Entfernung des Fällungsmittels und Klärung der Lösung wurde der so erhaltene Extrakt verschiedenen chromatographischen Reinigungsschritten unterworfen. Anstelle der Ausfällung der Proteine läßt sich der rohe VAC-Extrakt auch durch eine chromatographische Vorreinigung soweit reinigen, daß er anschließend einem Reinigungszyklus unterworfen werden kann. Als geeignetes Säulenmaterial für die Vorreinigung hat sich beispielsweise $SiO_2$ herausgestellt, doch sind auch andere Materialien mit ähnlichen Eigenschaften geeignet. Erfindungsgemäß wurde Silica Catalyst, Carrier, Grade 953 W der Firma Grace verwendet.

Ein für die Reinigung der erfindungsgemäßen Proteine geeigneter chromatographischer Reinigungszyklus bestand beispielsweise aus einer DEAE-Fast-Flow-Sepharose-, einer Sephacryl S-200 High Resolution- und einer Q-Sepharose-Fast-Flow-Chromatographie. Die Reinheit der so erhältlichen erfindungsgemäßen Proteine wurde mittels SDS-PAGE, Western Blot, Gelpermeations HPLC, Reverse HPLC und isoelektrischer Fokussierung bestimmt.

Bei der nach diesem Verfahren durchgeführten Lyse und Extraktion wurde das weiter zu reinigende Material in einer Ausbeute von ca. 50% erhalten.

Durch die Verbesserung dieses Verfahrensschrittes gemäß der vorliegenden Erfindung ist es überraschenderweise möglich, die Ausbeute nach diesem Schritt auf über 80% zu erhöhen.

Erreicht wurde diese Verbesserung überraschenderweise durch die Einstellung der homogenisierten Lyse-Suspension auf einen pH-Wert zwischen 8,0 und 10 vorzugsweise auf einen pH-Wert von 9,0. Besonders vorteilhaft wirkt sich die anschließende Zugabe von bivalenten Kationen, insbesondere von $Ca^{++}$ und von Phospholipiden wie Kephalin und Lecithin, insbesondere von Lecithin aus. Durch diese Maßnahme werden die in dem Homogenisat befindlichen VAC-Proteine an die Zellmembranen des Wirtsorganismus, vorzugsweise E.coli adsorbiert. Dieser in situ Adsorptionsschritt erlaubt überraschenderweise die Beseitigung störender löslicher Anteile aus dem Homogenisat durch einfaches Waschen. Die Desorption der an dem festen "Träger" gebundenen VAC-Proteine erfolgt mit Hilfe von chelatisierenden Agentien, vorzugsweise mit EDTA-haltigem Puffer.

Ein Aspekt der vorliegenden Erfindung besteht darin, ein Verfahren zur Reinigung von Annexinen bereitzustellen, dadurch gekennzeichnet,

a) daß der pH-Wert des Zellaufschluß-Homogenisates auf 8,0 bis 10,0 eingestellt wird,

b) daß mindestens ein bivalentes Kation aus der Gruppe $Ca^{2+}$, $Cd^{2+}$, $Zn^{2+}$, $Mn^{2+}$ und $Co^{2+}$ zugegeben wird,

c) daß ein Phospholipid zugegeben wird,

d) daß lösliche Bestandteile durch Waschen des Zellrückstandes entfernt werden und

e) daß die Proteine mit chelatisierenden Agentien aus dem Zellrückstand extrahiert werden.

Die Zugabe des Phospholipids (Stufe c) kann gleichzeitig oder stufenweise erfolgen.

Vorzugsweise kann eine Mischung bivalenter Kationen verwendet werden, beispielsweise $Ca^{2+}$ in Verbindung mit $Zn^{2+}$, wobei die molaren Verhältnisse jedes Kations so eingestellt werden, daß die Adsorption des Annexins, z.B. VAC, an der Zelloberfläche maximal ist.

Überraschenderweise ließ sich bei der Reinigung der Annexine die durch Expression in E. coli hergestellt worden waren, der Anteil der E. coli-Proteine (ECP) durch die Zugabe von BPA (Bioprocessing Aids, Fa. TOSOHAAS) deutlich reduzieren. Hierzu wurde dem nach der EDTA-Extraktion erhaltenen Rohextrakt BPA, beispielsweise BPA-1000, BPA-1050 oder BPA-2100, vorzugsweise BPA-1000 zugesetzt und gerührt. Die zuzugebende Menge kann 1 bis 20 Vol %, vorzugsweise 5 bis 10 Vol %, besonders bevorzugt 10 Vol % betragen. Der Niederschlag wird abzentrifugiert. Hieran schließen sich die Proteinfällung sowie Chromatographieschritte an. Durch die Zugabe von BPA ließ sich der Gehalt an ECP schon auf dieser Stufe auf bis zu 2 % des Ausgangswertes senken. Der Gehalt an VAC wurde dagegen nur um 6 % vermindert.

Eine weitere Ausbeuteverbesserung konnte überraschenderweise erreicht werden, indem die Zellen des Fermentationsansatzes vor dem Zellaufschluß inaktiviert wurden. Als Inaktivierungsmittel können einfache aromatische Verbindungen verwendet werden, unter anderem Benzol, Toluol, Phenol, Xylol oder Kresol. Besonders vorteilhaft ist m-Kresol zu verwenden.

Durch Vorschaltung dieses Inaktivierungsschrittes ließ sich in Verbindung mit dem weiter oben beschriebenen Verfahrensschritt die Ausbeute bei der Extraktion auf über 98 % steigern!

An den Adsorptions-/Desorptionsschritt schließen sich die aus der EPA 293 567 bekannten Reinigungsschritte an. Die hierzu erforderlichen Parameter wie Temperatur, Mengenverhältnisse, Reihenfolge der einzelnen Schritte, pH-Werte, besondere Reagentien etc. sind dem Fachmann bestens bekannt. Die unten angegebenen Beispiele können, falls gewünscht, in geeigneter, dem Fachmann bekannter Weise abgewandelt werden. Insbesondere stellen sie keine Einschränkung bezüglich des zu reinigenden Proteins dar. Aufgrund der gemeinsamen strukturellen Merkmale sowie der generellen Eigenschaften der Annexine, ist das erfindungsgemäße Verfahren auch anwendbar auf die Reinigung der übrigen Annexine, insbesondere ist es geeignet bei der Reinigung der Annexine IBC, PAP, PAP I, PP4, Endonexin, Lipocortin V und VAC.

Im übrigen ist das erfindungsgemäße Verfahren auch anwendbar bei der Isolierung und Reinigung von Annexinen aus stark vaskularisiertem Gewebe.

Die Angabe zu den verwendeten Reagenzien sind nur beispielhafte Herkunftsangaben und stellen keine Beschränkung dar.

## Legende zu den Figuren

Fig. 1:    Elutionsprofil zur Chromatographie an Phenyl-Sepharose Fast Flow ($\leftarrow \rightarrow$ = VAC-Pool)
Fig. 2:    Elutionsprofil zur Chromatographie an Q-Sepharose Fast Flow ($\leftarrow \rightarrow$ = VAC-Pool)

## Beispiel 1

### 1. ZELLAUFSCHLUSS:

#### Zugabe von $Ca^{++}$ and Lecithin, Waschen und Extraktion

122 g gefrorene, nicht inaktivierte Zellmasse des Klones E.coli HB101/pRH291 aus der Fermentation Nr. 524 wurden nach Auftauen mit 500 ml Lysispuffer durch Rühren suspendiert (ca. 1 Stunde, Eiskühlung) und anschließend mit dem Ultra-Turrax T 45/6 homogenisiert (ca. 1 Min., Eiskühlung).

LYSISPUFFER:

```
100 mM TRIS   12,14 g/1000 ml  Merck 8382
  1 mM EDTA    0,37 g/1000 ml  Titriplex III,
                               Merck 8418
200 mM NaCl   11,69 g/1000 ml  Merck 6404
```

Der pH-Wert wird mit 32%iger HCl auf 9,0 0,1 eingestellt.

Die Suspension wird 3-mal bei ca. 6000 psi durch eine Manton-Gaulin Presse Type 15 M 8TA homogenisiert, wobei das Auffanggefäß in Eis gekühlt wird. Der Apparat wird anschließend 2x mit jeweils 150-200 ml Lysispuffer nachgewaschen (totales Volumen des Extraktes: 1000 ml).

**2. Zugabe von $Ca^{++}$ und Lecithin zur Adsorption von VAC an den unlöslichen Zellrückstand.**

Zu dem Homogenat werden 0,55 g Calziumchlorid (Merck 2083) frisch gelöst - Endkonzentration 5 mM - und 6,1 ml Lecithin-Lösung (200 mg/ml Chloroform) zugegeben. Endkonzentration 1 g Lecithin/100 g Biomasse.

Das Gemisch wird 1 Min mit dem Ultra-Turrax homogenisiert und 60 Min im Eisbad gerührt.

LECITHIN: Lecithin aus Sojabohnen, pract., Serva 57556

Anschließend werden 110 ml 5% Polyminlösung (hergestellt aus 50% Polymin P, Serva 33141, verdünnt auf 5% und neutralisiert auf pH 8 mit 5 N HCl) zugegeben und nochmals 30 Min gerührt. Endkonzentration 0,5%. Abschließend wird die Suspension klarzentrifugiert (Beckman High-Speed Zentrifuge J2-21, Rotor JA 10, 8000 rpm, 30 Min., Einstellung 2°C).

ÜBERSTAND I:   1020 ml (4,25 mg Protein/ml; 0,05 mg VAC/ml).

**3. Waschen des Zellrückstandes zur Entfernung löslicher Anteile**

Der Zellrückstand wird mit 600 ml Waschpuffer mit Hilfe des Ultra-Turrax suspendiert und anschließend 30 Min gerührt.

WASCHPUFFER:

100 mM Tris
200 mM NaCl
5 mM $CaCl_2$

Der pH-Wert wird mit 32%iger HCl auf $9,0 \pm 0,1$ eingestellt.

Der Zellrückstand wird wie vorher durch Zentrifugieren gewonnen.

ÜBERSTAND II:   580 ml (1,35 mg Protein/ml; 0,04 mg VAC/ml)

Die Suspension in 350 ml Waschpuffer, die anschließende Homogenisation und das Rühren wird abermals wiederholt. Anschließend wird zentrifugiert.

ÜBERSTAND III:   340 ml (0,93 mg Protein/ml; 0,02 mg VAC/ml)

**4. Extraktion von VAC mit EDTA-haltigem Puffer**

Die Pellets werden mit 900 ml Extraktionspuffer im Ultra-Turrax homogenisiert und anschließend über Nacht im Kühlraum gerührt. Der Extrakt wird wie beschrieben zentrifugiert und die klare Lösung = ROHEXTRAKT gewonnen.

ROHEXTRAKT:   915 ml (1,98 mg Protein/ml; 0,65 mg VAC/ml)

EXTRAKTIONSPUFFER 20 Mm TRIS

50 mM   EDTA

Der pH-Wert wird mit 5 N NaOH auf 7,5 0,1 eingestellt.

ÜBERSICHT über die WASCHPROZEDUR und EXTRACKTION:

```
Überstand I      1020 ml    4335 mg Protein     49 mg VAC
Überstand II      580 ml     783 mg Protein     22 mg VAC
Überstand III     340 ml     316 mg Protein      5 mg VAC
                             5434 mg Protein     76 mg VAC (11%)


ROHEXTRAKT VAC 915 ml    1816 mg Protein      594 mg VAC
               in 122 g Zellmasse VAC total 670 mg (89%)
```

### Beispiel 2

## ÜBERSICHT:

1. Inaktivierung
2. Zellaufschluß
3. Zugabe von $Ca^{++}$ und Lecithin zur Adsorption von VAC an den unlöslichen Zellrückstand
4. Waschen des Zellrückstandes zur Entfernung löslicher Anteile
5. Extraktion von VAC mit EDTA-hältigem Puffer
6. Zugabe von 35% sat. Ammonsulfat und Entfernung des Niederschlages
7. Chromatographie an Phenyl-Sepharose Fast Flow
8. Dialyse des VAC-Pools und Chromatographie an Q-Sepharose Fast Flow
9. Konzentrierung des VAC-Pools und Chromatographie an Sephacryl S-200 HR

### 1. Inaktivierung

Ds Fermentationsende ist etwa nach 17 Stunden ab Phosphatverbrauch im Medium erreicht. Zu diesem Zeitpunkt werden 10 mM $CaCl_2$ zugesetzt und die Temperaturregelung auf eine möglichst rasche Abkühlung auf 5 - 8 Grad Celsius gestellt. Alle übrigen geregelten Parameter (wie Belüftung, Rührung, pH-Wert, Druck usw.) bleiben gleich. Nach ca. 10 Minuten werden 7 ml/1 m-Kresol zugegeben, anschließend Druck, Belüftung und pH-Regelung abgestellt und die Rührung auf ein Maß reduziert, das zwar eine gute Durchmischung der Fermentationsbrühe mit dem Kresol gewährleistet, die Schaumbildung aber begrenzt. Unter diesen Bedingungen wird mindestens 3, maximal 15 Stunden inaktiviert (Temperatur weiterhin 5 - 8 Grad Celsius).

### 2. Zellaufschluß

#### Zugabe von $Ca^{++}$ und Lecithin, Waschen und Extraktion

286 g gefrorene, mit m-Kresol inaktivierte Zellmasse des Klones HB 101/pGN25 werden nach Auftauen mit 750 ml Lysispuffer durch Rühren suspendiert (ca. 1 Stunde, Eiskühlung) und anschließend mit dem Ultraturrax T 45/6 homogenisiert (ca. 1 Min., Eiskühlung).

#### LYSISPUFFER:

100 mM TRIS
1 mM EDTA
200 mM NaCl
Der pH-Wert wird mit 32%iger HCl auf $9,0 \pm 0,1$ eingestellt.

Die Suspension wird 3-mal bei ca. 6000 psi durch eine Manton-Gaulin Presse Type 15 M 8TA homogenisiert, wobei das Auffanggefäß in Eis gekühlt wird. Der Apparat wird anschließend 2x mit jeweils 150-200 ml Lysispuffer nachgewaschen. Volumen des Homogenates: 1400 ml

**2. Zugabe von $Ca^{++}$ und Lecithin zur Adsorption von VAC an den unlöslichen Zellrückstand:**

Zu dem Homogenat werden 0,55 g $CaCl_2$ (Merck 2083)/1000 ml (Endkonzentration 5 mM) zugegeben (gelöst in ca. 10 ml $H_2O$) und 1 g Lecithin/100 g Biomasse, gelöst in Chloroform (ca. 0,2 g/ml), zugegeben.

Das Gemisch wird 1 Min mit dem Ultra-Turrax homogenisiert und 60 min im Eisbad gerührt.

LECITHIN: Lecithin aus Sojabohnen, pract., Serva 57556

Anschließend werden 0,11 Volumen 5% Polyminlösung (hergestellt aus 50% Polymin P, Serva 33141, verdünnt auf 5% und neutralisiert auf pH 8 mit 5 N HCl) zugegeben und nochmals 30 Min gerührt. Endkonzentration 0,5%. Anschließend wird die Suspension klarzentrifugiert (Beckman High-Speed Zentrifuge J2-21, Rotor JA 10, 8000 rpm, 30 Min, Einstellung 2°C).

ÜBERSTAND I: 1200 ml (5,8 mg Protein/ml; 0,01 mg VAC/ml)

**3. Waschen des Zellrückstandes zur Entfernung löslicher Anteile**

Der Zellrückstand wird mit ca. 1000 ml Waschpuffer mit Hilfe des Ultra-Turrax suspendiert und anschließend 30 Min gerührt.

WASCHPUFFER:

    100 mM Tris        200 mM NaCl        5 mM $CaCl_2$

Der pH-Wert wird mit 32%iger HCl auf $9,0 \pm 0,1$ eingestellt.

Der Zellrückstand wird wie vorher durch Zentrifugieren gewonnen.

ÜBERSTAND II: 1000 ml (1,94 mg Protein/ml; 0,01 mg VAC/ml)

Die Suspension in 1000 ml Waschpuffer mit anschließender Homogenisation und Rühren wird abermals wiederholt und anschließend zentrifugiert.

ÜBERSTAND III: 1000 ml (0,79 mg Protein/ml; 0,01 mg VAC/ml)

**4. Extraktion von VAC mit EDTA-haltigem Puffer**

Die Pellets werden mit 1350 ml Extraktionspuffer im Ultra-Turrax homogenisiert und anschließend über Nacht im Kühlraum gerührt. Der Extrakt wird wie beschrieben zentrifugiert und die klare Lösung = ROHEXTRAKT gewonnen.

ROHEXTRAKT: 1350 ml (3,35 mg Protein/ml; 1,46 mg VAC/ml

EXTRAKTIONSPUFFER: 20 mM TRIS

                          50 mM EDTA

Der pH-Wert wird mit 5 N NaOH auf 7,5 0,1 eingestellt.

## ÜBERSICHT über die WASCHPROZEDUR und EXTRAKTION:

```
Überstand I        1.200 ml    6.960 mg Protein    12 mg VAC

Überstand II       1.000 ml    1.940 mg Protein    10 mg VAC

Überstand III      1.000 ml      790 mg Protein    10 mg VAC
                               9.690 mg Protein    32 mg VAC (2%)


Rohextrakt VAC     1.350 ml    4.522 mg Protein    1.971 mg VAC

            in 286 g Zellmasse VAC total 2003 mg (98%)
```

### 5.-6. Fällung bei 35% sat. Ammonsulfat und Chromatographie an Phenyl-Sepharose Fast Flow

Fällung Ammonsulfat:

Zu dem Rohextrakt werden 209 g/l festes Ammonsulfat (Merck 1217) langsam unter Rühren zugegeben. Das ergibt einen Sättigungsgrad von 35%. Nach mindestens 1 Stunde Rühren im Kühlraum wird die Lösung klarzentrifugiert (Bedingungen wie vorher) und der Niederschlag verworfen. Die Lösung wird mit einer Geschwindigkeit von 8-10 ml/Min an der FPLC-Apparatur auf die vorbereitete Phenyl-Sepharose FF gepumpt.

Der klarzentrifugierte 35% sat. Ammonsulfat-Überstand wurde in 2 etwa gleichen Teilen weiterverarbeitet, und alle nachfolgenden Reinigungsschritte 2x nacheinander durchgeführt.

Vorbereitung der Säule:

Eine Pharmacia-Säule des Typs XK 26/40 (Bettvolumen ca. 200 ml) wird mit Phenyl-Sepharose Fast Flow (Pharmacia, Code No. 17-0965) gefüllt und mit Puffer A äqulibriert. Hierzu werden 2-3 CV (Column Volume) Puffer benötigt. Die Säule ist an einer FPLC-Apparatur angeschlossen. Die gesamte Chromatographie erfolgt bei Raumtemperatur.

PUFFER A:   50 mM TRIS

Der Puffer wird mit konz. HCl auf pH 7,2 ± 0,1 eingestellt.

Es werden sodann 209 g/1000 ml festes Ammonsulfat (Merck 1217) zugegeben.

PUFFER B:   Wie Puffer A, aber ohne Zugabe von Ammonsulfat.

Chromatographie:

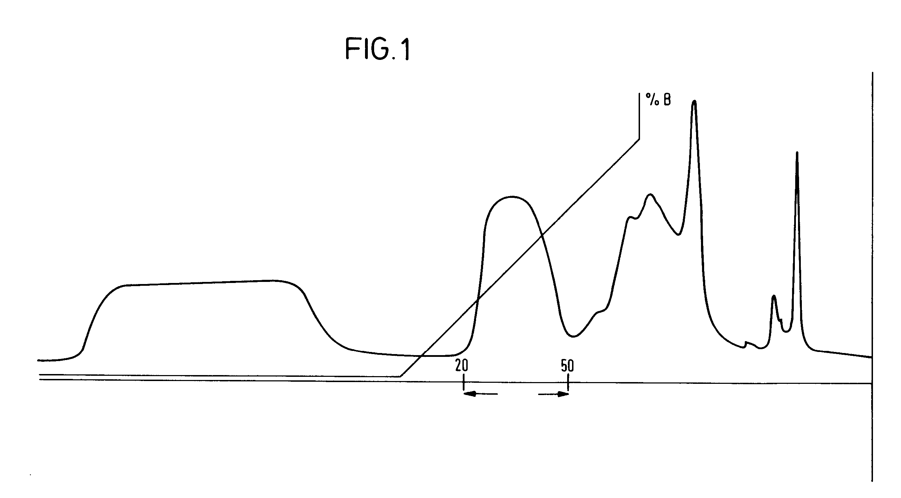

Das Eluat wird durch Messung der OD 280 nm kontrolliert. Sobald das Präparat auf die Säule aufgetragen ist, wird mit Puffer A solange nachgewaschen, bis die OD 280 nm auf den ursprünglichen Wert zurückgeht. Es wird dann ein Gradient Puffer A-Puffer B von 0-100% B von 5 Säulenvolumina (1000 ml) zur Elution des gebundenen VAC benützt. VAC ist der erste prominente Peak nach Beginn des Gradienten (siehe das Bild des Elutionsdiagrammes; Fig. 1). Es werden Aliquots des Durchlaufes, des VAC-Pools und eventuell noch weitere Peaks des OD-Profiles durch SDS-PAGE untersucht. Testung des VAC-Pools: Proteinbestimmung, VAC-Testung, SDS-PAGE.

Regenerieren der Säule:

Die Phenyl-Sepharose wird mit 2 Volumen 6 M Harnstoff regeneriert. Nach Auswaschen des Harnstoffes (1 CV aqua dest) wird sie in 24% Äthanol gelagert.

Zur Beseitigung bzw. Vermeidung gefärbter Bestandteile am oberen Ende der Säule wird ein erweitertes Wasch- und Regenerier-Verfahren verwendet:

1. 1 CV aqua dest
2. 2CV 6 M Harnstoff (Merck 8487)
3. 1CV aqua dest
4. 1CV 0,1 M NaOH
5. Waschen und Lagern 24% Äthanol
6. vor Gebrauch äquil. mit 3 CV Puffer A

### 7. Chromatographie an Q-Sepharose Fast Flow

Um bei der Dialyse des VAC-Pools gegen den Auftragepuffer der Q-Sepharose nicht allzuviel Puffer zu brauchen, wird der Pool zuerst in einer AMICON-Ultrafiltrationszelle und einem YM 10-Filter eingeengt: auf ca. 150 ml. Sodann wird gegen Puffer A dialysiert.

PUFFER A:   20 mM Bis-TRIS

Der pH-Wert wird mit 5 N HCl auf 6,3 $\pm$ 0,1 eingestellt.

PUFFER B:   Puffer A + 0,35 M NaCl

20 mM Bis-TRIS

350 mM NaCl

Der pH-Wert wird mit 5 N HCl auf 6,3 0,1 eingestellt.

Säulenvorbereitung:

Eine Säule Typ HR 16/50 Pharmacia (CV 100 ml) wird an das FPLC-System angeschlossen und mit der Füllvorrichtung mit Q-Sepharose Fast-Flow (Pharmacia) gefüllt. Sie wird mit 2 CV Puffer A äquilibriert. Die Chromatographie erfolgt bei Raumtemperatur.

Chromatographie:

Das Auftragen der Probe erfolgt mit 4 ml/Min. Es wird sodann mit Puffer A nachgewaschen, bis die OD 280 nm des Eluates auf den ursprünglichen Wert absinkt. Das Gradient-Programm ist wie folgt:

0,5 CV 0 - 30% B

8,0 CV 30 - 70% B (hier wird die Trennung ausgeführt)

ca. 1,0 CV 100% B

Waschprogramm:

Nachdem bei 100% B kein UV-aktiviertes Material mehr eluiert wird, beginnt das Waschprogramm:

2 CV aqua dest

2 CV 0,5 n NaOH

2 CV aqua dest

Storage: 24% Äthanol

vor dem Gebrauch wird mit 2-3 CV Puffer A

gewaschen: Kontrolle pH = 6,3

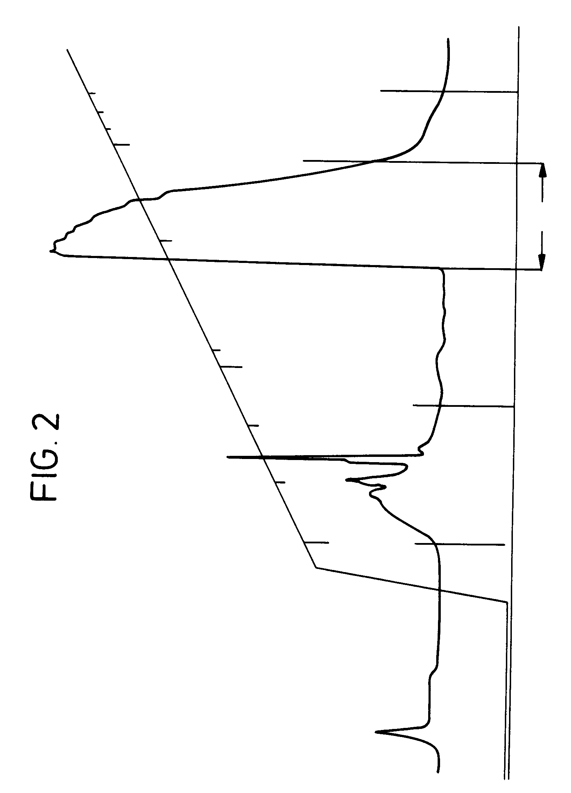

Die Verunreinigungen werden in dem Programm vor dem VAC eluiert. Das Elutionsprofil ist beigefügt (Fig. 2).

### 8. Chromatographie an SEPHACRYL S-200 HR

Der VAC-Pool wird mit einem AMICON-Ultrafilter YM 10 auf ca. 10 ml eingeengt. Dabei zeigte es sich, daß VAC bis zu 100 mg/ml (BioRad) ohne Probleme konzentriert werden kann.

Säulenvorbereitung:

Eine K 26//100 Säule von Pharmacia mit ca. 400 ml Bettvolumen wird nach Vorschrift mit Sephacryl S-200 High Resolution (Pharmacia) gefüllt und mit Elutionspuffer äquilibriert (2 CV).

ELUTIONSPUFFER:

20 mM Na-Phosphat pH = 7,2

150 mM NaCl oder

20 mM Bernsteinsäure $Na_2$-Salz (pH = 7,0)

0,01 % TWEEN 20

der pH-Wert wird mit 1N HCL auf

7,0 $\pm$ 0,1 eingestellt.

Chromatographie:

Die Chromatographie erfolgt im Kühlraum. Die Flußgeschwindigkeit beträgt 80 ml/Stunde. Der bei OD 280 nm aufgezeichnete Peak wird gesammelt. Das so gereinigte VAC war >99 % rein (RP-HPLC Analyse).

Regenerieren der Sephacryl-Säule:

Waschprogramm:
1 CV aqua dest
2 CV 0,5 N NaOH (+ Lagerung über einige Tage)
(Bei längerer Lagerung empfiehlt sich: 1 M NaCl + 0,0025% NaOH)
Regenerieren:
1 CV aqua dest
2 CV Elutionspuffer

Übersicht über die gesamte Reinigung

| Reinigungsstufe | Volumen (ml) | Protein[x] (mg) | VAC-alpha[xx] (mg) |
|---|---|---|---|
| Überstand I | 1.200 | 6.960 | 12 |
| Überstand II | 1.000 | 1.940 | 10 |
| Überstand III | 1.000 | 790 | 10 |
| Rohextrakt | 1.350 | 4.522 | 1.971 |
| Überstand 35% sat. $Am_2SO_4$ | 1.500 | 4.500 | 2.098 |
| Phenyl-Seph.FF-Pool | 975 | 3.050 | 2.041 |
| Load Q-Seph.FF | 355 | 3.062 | 2.277 |
| Q-Seph.FF-Pool | 475 | 2.660 | 2.082 |
| Load Seph.S-200 HR | 23,7 | 2.446 (1.859)[xxx] | n.b. |
| Seph.S-200 HR-Pool | 192 | 2.477 (1.883) | 2.126 |

x) Biorad Protein-Assay (Standard: Rinderserum Albumin)

**xx)** VAC-alpha-Assay: Hemmung der Bildung von Thrombin aus Pro-Thrombin (Reutelingsperger, Hornstra und Hemker, Eur.J.Biochem. 151, 625-629; 1985). Als Referenz diente ein gereinigtes VAC-alpha Präparat.

**xxx)** Proteingehalt bestimmt durch Messung der O.D. 280 nm unter Verwendung des für reines VAC-alpha bestimmten Faktors: O.D. 280 nm x 1,277 = mg/ml VAC-alpha

Beispiel 3

**Reinigung des EDTA-Rohextraktes mit BPA**

Die in Beispiel 1 und Beispiel 2 beschriebene Reinigung wurde bis zur Extraktion mit EDTA-haltigem Puffer durchgeführt (Stufe 4). Ausgangsmaterial waren einmal 295 g und einmal 240 g Biomasse:

Nach Gewinnung des Rohextraktes wurden 10 Vol. % BPA 1000 zugegeben und 10 Min. gerührt. Der entstandene Niederschlag wurde abzentrifugiert (High-Speed Zentrifuge J2-21 Fa. Beckman, Rotor JA 10, 8000 rpm, 30 Min. bei 2°C).

Die weitere Reinigung erfolgte wie beschrieben: Zugabe von festem Ammonsulfat zu 35 % Sättigung und weitere Chromatographie an Phenyl-Sepharose Fast Flow (siehe die vorstehende Vorschrift).

Ergebnisse für VAC alpha

Ausgangmaterial waren 295 g Biomasse (Charge B005001)

| Stufe: | Vol. ml | Protein mg/ml | VACalpha mg/ml (Total) | ECP ppm |
|---|---|---|---|---|
| Rohextrakt | 700 | 4,0 | 1,87 (1309) | 126.000 |
| Nach BPA 1000 | 700 | 2,7 | 1,77 (1239) | 11.700 |
| Phenyl-Sepharose Fast Flow Pool | 215 | 3,23 | 4,23 ( 909) | not done |
| Q-Sepharose Fast Flow Pool | 169 | 3,83 | 4,70 ( 794) | 39 |
| Sephacryl S-200 HR Pool (Endprodukt) | 59,7 | 10,4 | 10,9 ( 651) | 41 |

Ergebnisse für VAC alpha

Ausgangmaterial waren 240 g Biomasse (Charge B005001)

| Stufe: | Vol. ml | Protein mg/ml | VACalpha mg/ml (Total) | ECP ppm |
|---|---|---|---|---|
| Rohextrakt | 680 | 3,20 | 1,58 (1074) | 776.000 |
| Nach BPA 1000 | 680 | 2,00 | 0,95 ( 646) | 19.600 |
| Phenyl-Sepharose Fast Flow Pool | 205 | 3,73 | 2,86 ( 586) | 2.370 |
| Q-Sepharose Fast Flow Pool | 150 | 4,55 | 3,40 ( 510) | 45 |
| Sephacryl S-200 HR Pool (Endprodukt) | 56,0 | 7,10 | 7,40 ( 414) | 35 |

**Patentansprüche**

1. Verfahren zur Reinigung von Annexinen, dadurch gekennzeichnet,
   a) daß der pH-Wert des Zellaufschluß-Homogenisates auf 8,0 bis 10,0 eingestellt wird,
   b) daß mindestens ein bivalentes Kation aus der Gruppe $Ca^{2+}$, $Cd^{2+}$, $Zn^{2+}$, $Mn^{2+}$ und $Co^{2+}$ zugegeben wird,
   c) daß ein Phospholipid zugegeben wird,
   d) daß lösliche Bestandteile durch Waschen des Zellrückstandes entfernt werden und
   e) daß die Proteine mit chelatisierenden Agentien aus dem Zellrückstand extrahiert werden.

2. Verfahren nach Anspruch 1, dadurch gekennzeichnet, daß im Anschluß an Schritt e) BPA (Bioprocessing Aids), vorzugsweise BPA 1000 zugegeben und der entstandene Niederschlag abgetrennt wird.

3. Verfahren nach Anspruch 1 oder 2, dadurch gekennzeichnet, daß im Anschluß an Schritt e) die extrahierten Proteine durch Proteinfällung und anschließender Proteinreinigungsschritte in an sich bekannter Weise weiter gereinigt werden.

4. Verfahren nach einem der Ansprüche 1 bis 3, dadurch gekennzeichnet, daß der pH-Wert auf 9,0 eingestellt wird.

5. Verfahren nach einem der Ansprüche 1 bis 4, dadurch gekennzeichnet, daß als bivalentes Kation $Ca^{2+}$ und/oder $Zn^{2+}$ zugegeben wird.

6. Verfahren nach einem der vorhergehenden Ansprüche, dadurch gekennzeichnet, daß als Phospholipid Lecithin zugegeben wird.

7. Verfahren nach einem der vorhergehenden Ansprüche, dadurch gekennzeichnet, daß die Schritte b) und c) praktisch gleichzeitig ausgeführt werden.

8. Verfahren nach einem der vorhergehenden Ansprüche, dadurch gekennzeichnet, daß als chelatisierendes Agenz EDTA verwendet wird.

9. Verfahren nach einem der vorhergehenden Ansprüche, dadurch gekennzeichnet, daß die Zellen vorher inaktiviert werden.

10. Verfahren nach Anspruch 9, dadurch gekennzeichnet, daß als Inaktivierungsmittel einfache aromatische Verbindungen, insbesondere Benzol, Toluol, Phenol, Xylol oder Kresol, besonders bevorzugt Kresol verwendet wird.

**11.** Verfahren nach Anspruch 9, dadurch gekennzeichnet, daß als Inaktivierungsmittel m-Kresol verwendet wird.

**12.** Verfahren nach einem der vorhergehenden Ansprüche, dadurch gekennzeichnet, daß das zu reinigende Protein das VAC (vascular anticoagulating protein) oder seine Analoga ist.

## Claims

**1.** Process for purifying annexines, characterized in that
a) the pH value of the homogenised cell breakdown material is adjusted to 8.0 to 10.0,
b) at least one bivalent cation selected from the group $Ca^{2+}$, $Cd^{2+}$, $Zn^{2+}$, $Mn^{2+}$ and $Co^{2+}$ is added,
c) a phospholipid is added,
d) soluble constituents are removed by washing the cell residue, and
e) the proteins are extracted from the cell residue with chelating agents.

**2.** Process according to claim 1, characterized in that, following step e), BPA (Bioprocessing Aids), preferably BPA 1000 is added and the resulting precipitate is separated off.

**3.** Process according to claim 1 or 2, characterized in that, following step e), the extracted proteins are further purified by protein precipitation and subsequent protein purifying steps in a manner known per se.

**4.** Process according to claims 1 to 3, characterized in that the pH is adjusted to 9.0.

**5.** Process according to claims 1 to 4, characterized in that $Ca^{2+}$ and/or $Zn^{2+}$ is added as bivalent cation.

**6.** Process according to one of the preceding claims, characterized in that lecithin is added as the phospholipid.

**7.** Process according to one of the preceding claims, characterised in that steps b) and c) are carried out substantially simultaneously.

**8.** Process according to one of the preceding claims, characterized in that EDTA is used as the chelating agent.

**9.** Process according to one of the preceding claims, characterized in that the cells are inactivated beforehand.

**10.** Process according to claim 9, characterised in that simple aromatic compounds, particularly benzene, toluene, phenol, xylene or cresol, but particularly cresol, are used as the deactivating agents.

**11.** Process according to claim 9, characterized in that m-cresol is used as the deactivating agent.

**12.** Process according to one of the preceding claims, characterized in that the protein to be purified is VAC (vascular anticoagulating protein) or an analogue thereof.

## Revendications

**1.** Procédé pour purifier les annexines, caractérisé en ce que
a) le pH de l'homogénat de lyse des cellules est ajusté entre 8,0 et 10,0,
b) au moins un cation bivalent du groupe formé par $Ca^{2+}$, $Cd^{2+}$, $Zn^{2+}$, $Mn^{2+}$ et $Co^{2+}$ est ajouté,
c) un phospholipide est ajouté,
d) les constituants solubles sont éliminés par lavage du résidu cellulaire et
e) les protéines sont extraites du résidu cellulaire avec des agents chélatants.

**2.** Procédé selon la revendication 1, caractérisé en ce qu'à la suite de l'étape e) des BPA (Bioprocessing Aids), de préférence le BPA 1000, sont ajoutés et le précipité formé est séparé.

**3.** Procédé selon la revendication 1 ou 2, caractérisé en ce qu'à la suite de l'étape e) les protéines extraites sont purifiées encore de manière connue en soi par précipitation des protéines suivie par des étapes de purification des protéines.

4. Procédé selon l'une des revendications 1 à 3, caractérisé en ce que le pH est ajusté à 9,0.

5. Procédé selon l'une des revendications 1 à 4, caractérisé en ce que l'on ajoute comme cation bivalent $Ca^{2+}$ et/ou $Zn^{2+}$.

6. Procédé selon l'une des revendications précédentes, caractérisé en ce que l'on ajoute une lécithine comme phospholipide.

7. Procédé selon l'une des revendications précédentes, caractérisé en ce que les étapes b) et c) sont accomplies pratiquement en même temps.

8. Procédé selon l'une des revendications précédentes, caractérisé en ce que l'on utilise EDTA comme agent chélatant.

9. Procédé selon l'une des revendications précédentes, caractérisé en ce que les cellules sont inactivées au préalable.

10. Procédé selon la revendication 9, caractérisé en ce que l'on utilise comme agents d'inactivation des composés aromatiques simples, en particulier le benzène, le toluène, le phénol, le xylène ou le crésol, de manière particulièrement préférée le crésol.

11. Procédé selon la revendication 9, caractérisé en ce que l'on utilise le m-crésol comme agent d'inactivation.

12. Procédé selon l'une des revendications précédentes, caractérisé en ce que la protéine à purifier est la VAC (vascular anticoagulating protein) ou ses analogues.

FIG.1

% B

EP 0 409 053 B1

FIG. 2